# EUROPEAN PATENT APPLICATION

(11) **EP 4 389 265 A1**
(43) Date of publication of application: **26.06.2024**
(21) Application number: 22215058.3
(22) Date of filing: 20.12.2022
(51) Int. Cl.: B01D 63/08, B01D 67/00, B01D 69/10, B01D 65/00, B01L 3/00, G01N 33/49

(54) **MICROFLUIDIC DEVICE MOUNTED WITH MEMBRANE FILTER WITH ATTACHED SALT AND APPARATUS FOR MOUNTING MEMBRANE FILTER**

(71) Applicant: 1Drop Inc., Gyeonggi-do 13217 (KR)
(72) Inventor: KIM, Kyung Hak, 13351 Seongnam (KR); KIM, Won Jung, 13523 Seongnam (KR); BAEK, So Hyeon, 13351 Seongnam (KR); RHEE, Joo Won, 13562 Seongnam (KR)
(74) Representative: advotec.

(57) **Abstract**

The present disclosure is to provide a method of mounting a membrane filter and a microfluidic device including the membrane filter, which are capable of increasing a flow pressure of a fluid. A salt matrix is attached to a region of a surface of the membrane filter that adjoins the filter support structure and is exposed toward the movement passage. A membrane filter and a microfluidic device including the membrane filter, which are capable of increasing a flow pressure of a fluid.

## Description

### BACKGROUND

### Field

The present disclosure relates to a biosensor strip, and more particularly, to a method of allowing a specimen such as blood, urine, or saliva to pass through a membrane filter and allowing a desired component to flow to a channel.

### Description of the Related Art

The contents disclosed in this section only provide background information in respect to the embodiment disclosed in the present specification but do not necessarily constitute the prior art.

A measurement method, which uses a sample collection device, i.e., a sensor strip (or a biosensor strip), is used to examine a particular characteristic value by using a specimen such as blood, urine, or saliva. For example, to measure blood glucose, the sensor strip is inserted into an input port of a blood glucose measurer, blood is gathered from an end of a finger, and a small amount of gathered blood is brought into contact with the sensor strip inserted into the measurer. In this case, the blood is automatically introduced into the input port of the sensor strip, and a result value is displayed on a screen of the blood glucose measurer.

In this case, a membrane filter is used to filter out blood cell components, such as red blood cells and white blood cells, from the blood and allows only plasma components to pass therethrough. However, a flow pressure of a fluid becomes lower than a pressure spike when the blood passes through the membrane filter. The pressure spike means that an aqueous fluid is not introduced when a material of the membrane is hydrophobic, or the fluid does not flow away from the membrane when a material of the membrane is hydrophilic. The pressure spike occurs on a contact surface between the membrane and air or between the fluid and air.

In the related art, a pump is used to generate a positive or negative pressure to solve a problem that the flow pressure of the fluid becomes lower than the pressure spike. However, the method using the pump basically causes a problem that costs of the sensor strip are increased, blood cells are damaged by a pressure, and quality of plasma deteriorates. Another technology in the related art is a method of wetting in advance a membrane with a solvent such as ethanol or acetone. This method does not cause a problem of damage to plasma. However, there is a problem in that a sealed container is required to prevent the evaporation of the solvent, costs are increased, and a storage period is decreased.

### [Document of Related Art]

### [Patent Document]

(Patent Document 1) Korean Patent No. 10-1062356

### SUMMARY

An object to be achieved by the present disclosure is to provide a method of mounting a membrane filter and a microfluidic device including the membrane filter, which are capable of increasing a flow pressure of a fluid.

The present specification is not limited to the aforementioned technical problems, and other technical problems, which are not mentioned above, may be clearly understood by those skilled in the art from the following descriptions.

According to an aspect of the present disclosure, a method of mounting a membrane filter includes: (a) providing a two-sided bonding member having a bonding matrix with a bonding force at two opposite surfaces thereof and protective film attached on one of the two opposite surfaces; (b) attaching the two-sided bonding member to one surface of a membrane filter having desired filtering properties, in a state in which a partial region of one surface of the membrane filter to which the two-sided bonding member is attached is exposed; (c) applying salt onto one surface of the membrane filter to which the two-sided bonding member is attached; (d) removing the protective film of the two-sided bonding member attached to the membrane filter; and (e) aligning the membrane filter so that the two-sided bonding member, on which a bonding agent exposed from the removed protection film, corresponds to a filter support structure, and bonding the membrane filter to the filter support structure.

According to the embodiment of the present disclosure, a shape of a non-exposed region of one surface of the membrane filter to which the two-sided bonding member is attached may correspond to a shape of the filter support structure.

According to the embodiment of the present disclosure, in the two-sided bonding member provided in (a), a shape of the bonding matrix may correspond to a shape of the filter support structure.

According to the embodiment of the present disclosure, (b) may be a step of attaching the two-sided bonding member to a region of one surface of the membrane filter that corresponds to a shape of the filter support structure.

According to the embodiment of the present disclosure, in (b), an area of an exposed region of one surface of the membrane filter may be smaller than an area of an upper portion of the filter support structure.

According to the embodiment of the present disclosure, (c) may further include applying the salt by using a method of diffusing an intermediate medium and drying the salt.

According to another aspect of the present disclosure, a microfluidic device includes: a microfluid channel provided in a body; a sample input port formed in the body and provided in a region adjacent to one end of the microfluid channel; a reaction chamber formed in the body and connected to the other end of the microfluid channel; a membrane filter disposed between the sample input port and the microfluid channel and having desired filtering properties; a filter support structure provided between the membrane filter and the microfluid channel and configured to support the membrane filter, the filter support structure having one or more protrusion patterns that define movement passages so that a fluid separated from a sample injected through the sample input port is collected in the microfluid channel; and a bonding member disposed between the membrane filter and upper surfaces of the one or more protrusion patterns, in which a salt matrix is attached to a region of a surface of the membrane filter that adjoins the filter support structure and is exposed toward the movement passage.

Other detailed matters of the present disclosure are included in the detailed description and the drawings.

According to the present specification, it is possible to prevent the flow pressure of the fluid from temporarily becoming lower than the pressure spike. Therefore, the fluid passing through the membrane filter may more smoothly flow to the microfluid channel, such that the sufficient test may be performed even by using a small quantity of samples.

The effects of the present disclosure are not limited to the aforementioned effects, and other effects, which are not mentioned above, will be clearly understood by those skilled in the art from the following description.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 is an exploded perspective view for assisting in understanding a microfluidic device according to the present specification;
FIG. 2 is a cross-sectional view illustrating a membrane filter and a filter support structure;
FIGS. 3 to 9 are reference views for explaining a method of mounting the membrane filter according to the present specification;
FIGS. 10 to 17 are views illustrating other embodiments related to the microfluidic device and the method of mounting the membrane filter according to the present specification; and
FIG. 18 is a view illustrating various embodiments of a protrusion pattern.

### DETAILED DESCRIPTION OF THE EMBODIMENT

Advantages and features disclosed in the present specification and methods of achieving the advantages and features will be clear with reference to embodiments described in detail below together with the accompanying drawings. However, the present specification is not limited to the exemplary embodiments disclosed herein but will be implemented in various forms. The exemplary embodiments of the present specification are provided so that the present specification is completely disclosed, and a person with ordinary skill in the art can fully understand the scope of the present specification. The protection scope of the present specification will be defined only by the scope of the appended claims.

The terms used in the present specification are for explaining the exemplary embodiments, not for limiting the protection scope of the present specification. Unless particularly stated otherwise in the present specification, a singular form also includes a plural form. The term "comprise" and/or "comprising" used in the specification does not exclude existence or addition of one or more other constituent elements in addition to the mentioned constituent element.

Like reference denotations refer to like elements throughout the specification. As used herein, the term "and/or" includes each and all combinations of one or more of the mentioned components. Terms "first", "second", and the like may be used to describe various constituent elements, but the constituent elements are of course not limited by these terms. These terms are merely used to distinguish one constituent element from another constituent element. Therefore, the first constituent element mentioned hereinafter may of course be the second constituent element within the technical spirit of the present disclosure.

Unless otherwise defined, all terms (including technical and scientific terms) used in the present specification may be used as the meaning which may be commonly understood by the person with ordinary skill in the art to which the present specification pertains. In addition, terms defined in a generally used dictionary shall not be construed in ideal or excessively formal meanings unless they are clearly and specially defined in the present specification.

Spatially relative terms, such as "below," "beneath," "lower," "above," "upper," and the like, may be used herein for ease of description of one constituent element or a correlation between one constituent element and other constituent elements, as illustrated in the drawings. It should be understood that the spatially relative terms encompass different orientations of the constituent elements in use or operation in addition to the orientation depicted in the drawings. For example, if the constituent element in the drawings is turned over, the constituent element described as being "below" or "beneath" the other constituent element may be placed "above" the other constituent element. Thus, the exemplary term "below" can encompass both orientations of above and below. The constituent elements may be oriented in different directions, and the spatially relative terms used herein may be interpreted in accordance with the orientations. Hereinafter, embodiments of the present disclosure will be described in detail with reference to the accompanying drawings.

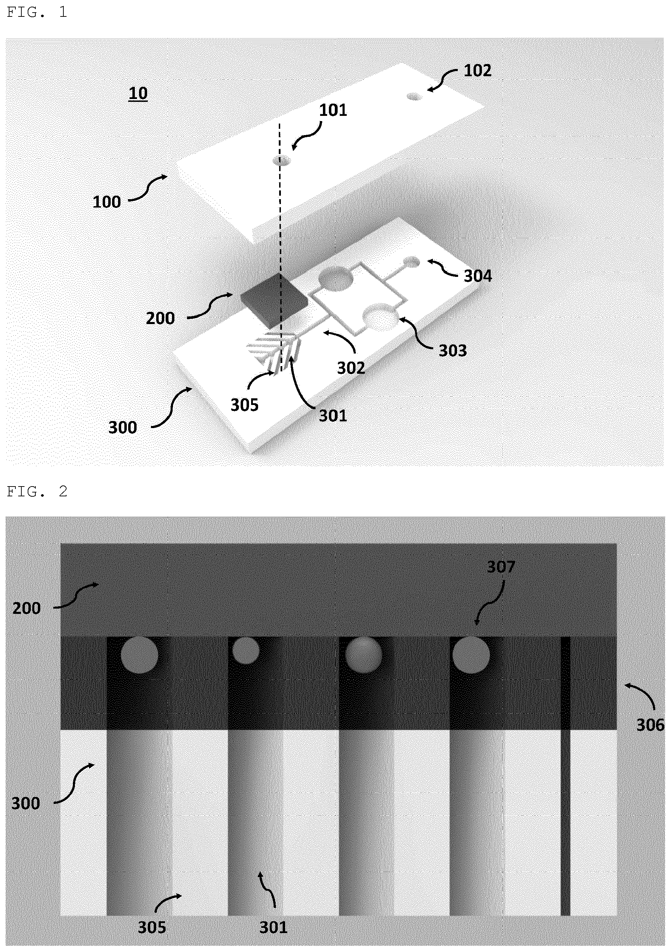

FIG. 1 is an exploded perspective view for assisting in understanding a microfluidic device according to the present specification.

Referring to FIG. 1, a microfluidic device 10 according to the present specification may include an upper housing 100, a membrane filter 200, and a lower housing 300. The upper and lower housings 100 and 300 may be made of metal or polymer synthetic resin. The upper and lower housings 100 and 300 may define a body by engaging with each other or being coupled by a bonding agent. In this case, the body has a sealed structure therein. An input port 101 and a discharge port 102 may be formed at an upper end of the upper housing 100 and formed through the upper housing 100. The membrane filter 200 may be disposed in a space below the input port 101 when the upper housing 100 and the lower housing 300 are coupled. To this end, the upper housing 100 and/or the lower housing 300 may have a space in which the membrane filter 200 may be seated. A flow path (hereinafter, referred to as a 'microfluid channel') may be formed in an upper surface of each of the upper housing 100 and/or the lower housing 300, and a microfluid flows along the microfluid channel. A plurality of sub-channels 301 may be formed in the space in which the membrane filter 200 is seated, such that the fluid passing through the membrane filter 200 may be collected at a center of the space. The plurality of sub-channels 301 may be connected to a main channel 302 formed at the center, and the main channel may be connected to a reaction chamber 303. A reaction reagent may be applied in advance in the reaction chamber 303. A result of a reaction of a sample and the reagent in the reaction chamber 303 may be detected from the outside. The reaction chamber 303 may be connected to a discharge channel 304. An end of the discharge channel 304 may be connected to the discharge port 102 formed in the upper housing 100.

The example illustrated in FIG. 1 is just one example for assisting in understanding the microfluidic device 10 according to the present specification. Therefore, the body 100 and 200 may be made of various materials and have various structures and shapes. In addition, various areas, shapes, connection points, and the like of the microfluid channels 301 to 304 may also be implemented. Various sizes and shapes of the reaction chamber 303 may also be implemented, and the number of reaction chambers 303 may also be variously implemented. The microfluidic device 10 according to the present specification includes the microfluid channels 301 to 304, a sample input port 101, the reaction chamber 303, the membrane filter 200, and a filter support structure 305 that are main components provided in the body. The sample input port 101 may be formed in the body and disposed in a region adjacent to one end of each of the microfluid channels 301 to 304. Therefore, the sample input port 101 may be similar to the input port illustrated in FIG. 1 but need not be necessarily formed in the upper housing 100. The reaction chamber 303 may be formed in the body and connected to the other end of each of the microfluid channels 301 to 304. The membrane filter 200 may be disposed between the sample input port 101 and the microfluid channels 301 to 304 and have desired filtering properties. The membrane filter 200 serves to separate a desired component from a specimen, and various filters widely known to those skilled in the art may be applied. For example, the description of the membrane filter 200 may be replaced with the contents disclosed in Korean Patent No. 10-2079783 filed by the present applicant.

The filter support structure 305 may be provided between the membrane filter and the microfluid channel and serve to support the membrane filter 200. In this case, the filter support structure 305 may have one or more protrusion patterns that define movement passages so that the fluid separated from the sample injected through the sample input port 101 is collected in the microfluid channels 301 to 304. The movement passages defined by the filter support structure 305 may be the sub-channels 301 illustrated in FIG. 1.

FIG. 2 is a cross-sectional view illustrating the membrane filter and the filter support structure.

Referring to FIG. 2, it can be seen that the membrane filter 200 is positioned at an upper end of the filter support structure 305. Because the filter support structure 305 may have the one or more protrusion patterns 305, the membrane filter 200 may be seated on upper surfaces of the one or more protrusion patterns 305. In this case, a bonding member 306 may be disposed between the membrane filter and the upper surfaces of the one or more protrusion patterns. The bonding member 306 serves to fix the membrane filter 200 to the upper end of the filter support structure 305. It is apparent that the bonding member 306 may be made of various materials known to those skilled in the art.

Meanwhile, the one or more protrusion patterns of the filter support structure 305 define the movement passages, and the membrane filter 200 has a surface that adjoins the filter support structure and has a region exposed toward the movement passages. A salt matrix 307 is attached to the exposed region.

The salt matrix 307 contains fine or nanoparticle soluble substances that generate hygroscopicity and osmotic force implemented by a pore structure generated by sizes of particles and spaces between the particles and chemical compositions of the particles. Examples of the salt matrix may include sodium chloride (NaCl), low-viscosity cellulose salt, anhydrous acid (e.g., ethylenediaminetetraacetic acid (EDTA)), sugar (e.g., sucrose or dextrose), sugar derivatives (e.g., heparin), and the like. The salt is disposed below the membrane filter 200 and has higher capillary force than the membrane filter, thereby increasing the flow pressure of the fluid so that the flow pressure of the fluid is higher than the pressure spike. Therefore, it is possible to eliminate a phenomenon in which the pressure spike, which is generated on a contact surface between the membrane and air or between the fluid and air, becomes higher than the flow pressure of the fluid. Meanwhile, the salt matrix may be water soluble and dissolved in the fluid. However, the salt matrix may remove or minimize the influence of measurement by performing the measurement in consideration of the presence of the salt during the separation process or reaction before the reaction between the reagent and the sample.

In the present specification, the salt matrix 307 is attached to a lower surface of the membrane filter 200. However, the salt matrix 307 is attached only to the region of the lower surface of the membrane filter 200 exposed toward the movement passages 301 without being attached to the region between the membrane filter 200 and the filter support structure 305. Hereinafter, according to the above-mentioned characteristics, a method of attaching the salt only to a particular region and mounting the membrane filter 200 with the attached salt will be described.

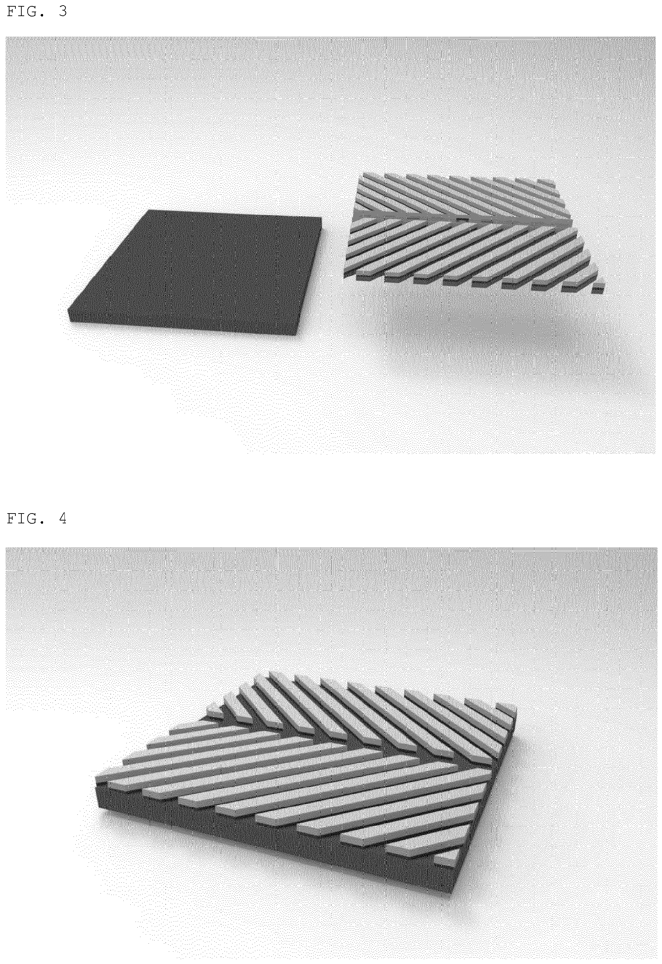

FIGS. 3 to 9 are reference views for explaining a method of mounting the membrane filter according to the present specification.

Referring to FIG. 3, the membrane filter 200 and the two-sided bonding member 306 may be provided. The two-sided bonding member 306 may be in a state in which bonding matrices having a bonding force are attached to two opposite surfaces of the two-sided bonding member 306, and a protective film is attached to one of the two opposite surfaces having the bonding force.

Referring to FIG. 4, the two-sided bonding member 306 may be attached to one surface of the membrane filter 200. In this case, as illustrated in FIG. 4, a partial region of one surface of the membrane filter to which the two-sided bonding member 306 is attached may be exposed. Further, a shape of a non-exposed region of one surface of the membrane filter to which the two-sided bonding member 306 is attached may correspond to a shape of the filter support structure 305.

Meanwhile, the example illustrated in FIG. 3 is an example in which a shape of the bonding matrix of the two-sided bonding member 306 corresponds to a shape of the filter support structure 305. That is, the example illustrated in FIG. 3 is an example in which the bonding matrix of the two-sided bonding member 306 has in advance a shape corresponding to the filter support structure 305 before the two-sided bonding member 306 is bonded to the membrane filter 200. However, according to another embodiment, the two-sided bonding member 306 may be attached only to the region of one surface of the membrane filter 200 that corresponds to the shape of the filter support structure 305.

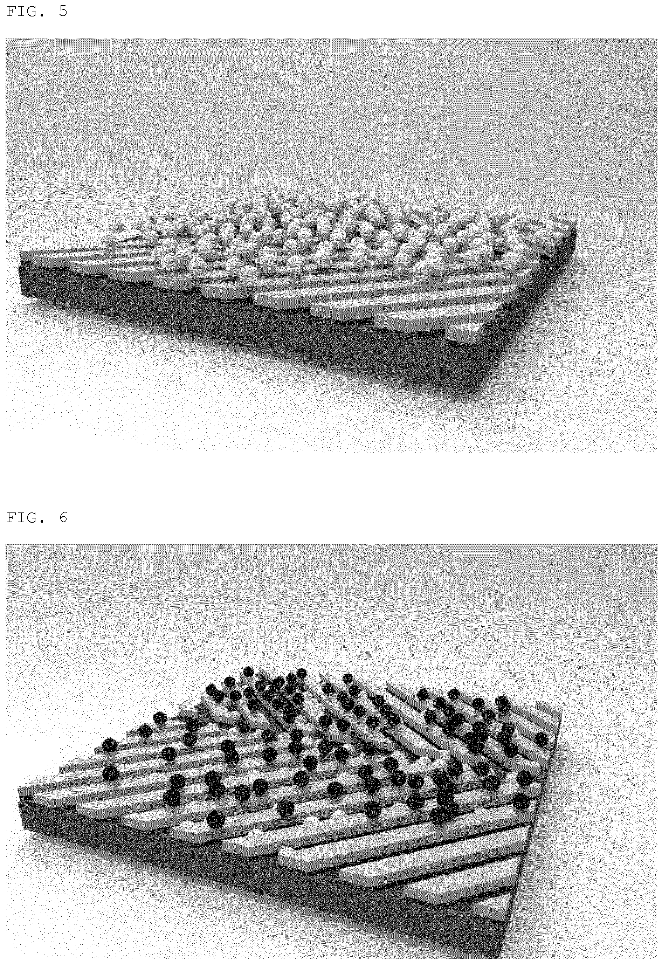

Next, referring to FIG. 5, the salt may be applied onto one surface of the membrane filter 200 to which the two-sided bonding member 306 is attached. The salt is a substance of the above-mentioned salt matrix. In this case, the applied salt is in direct contact with the exposed region of the membrane filter 200, and the applied salt in the non-exposed region is in contact with the two-sided bonding member 306. Referring to FIG. 6, a portion in a light blue (sky-blue) color indicates the salt applied in the exposed region of the membrane filter 200, and a portion in a dark blue color indicates the salt applied onto the two-sided bonding member 306.

Meanwhile, the salt may be applied by a method of diffusing an intermediate medium such as mist, spray, or dipping aerosol and then be dried. After the drying process, the salt may define a matrix by being attached to the surface of the membrane filter 200.

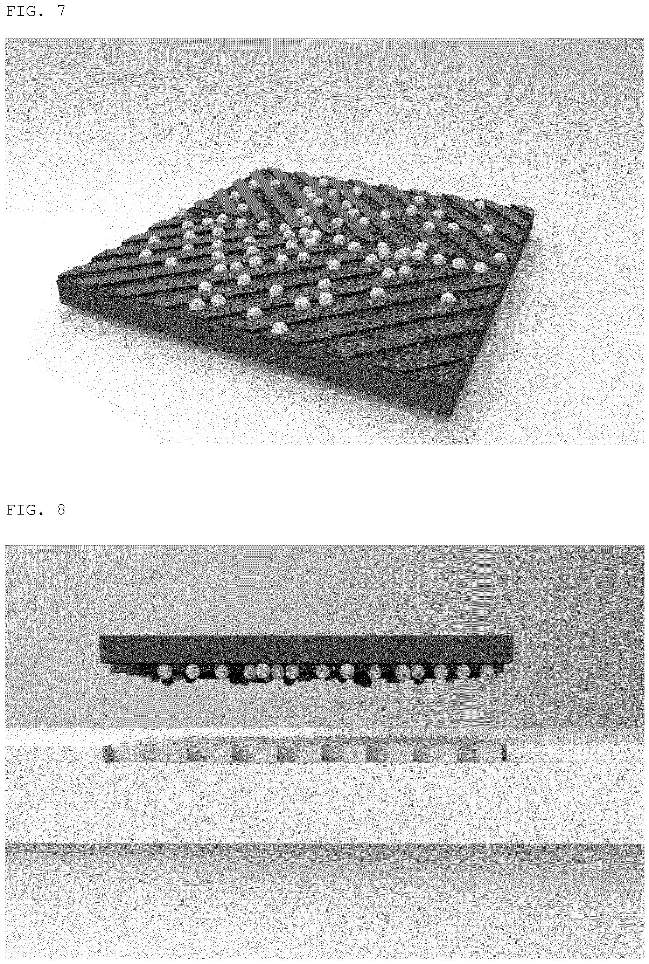

Next, referring to FIG. 7, the protective film of the two-sided bonding member 306 attached to the membrane filter 200 may be removed. Therefore, the salt applied onto the two-sided bonding member 306 is removed, and the salt matrix applied onto the membrane filter 200 remains.

Next, referring to FIG. 8, the membrane filter 200 is aligned so that the two-sided bonding member 306, on which the bonding agent is exposed as the protective film is removed, corresponds to the filter support structure, and then the membrane filter 200 may be bonded to the filter support structure.

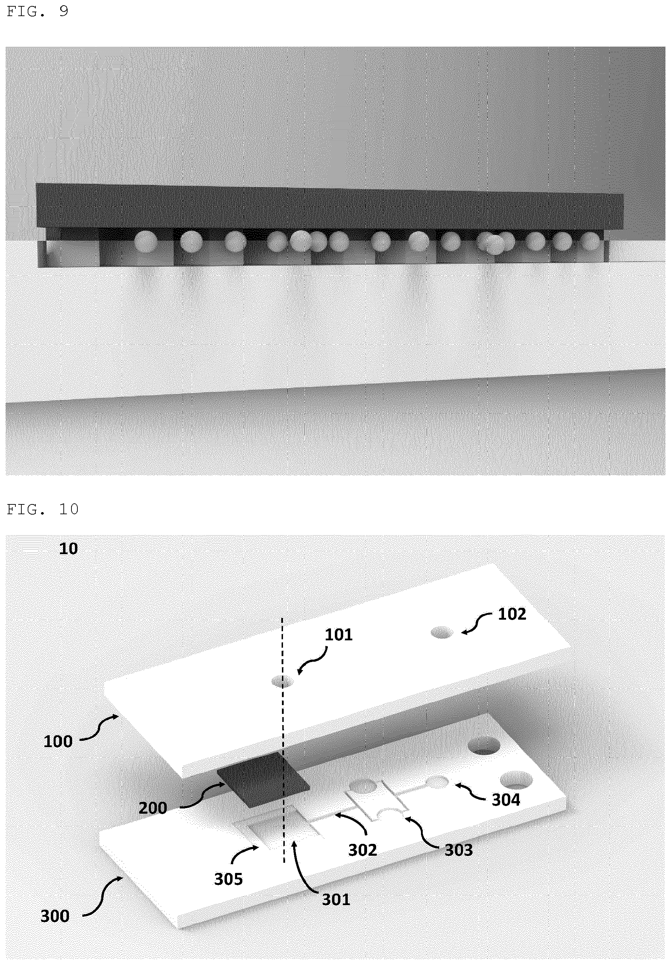

Finally, referring to FIG. 9, the membrane filter 200 may be mounted on the microfluidic device 10 in a state in which the salt matrix 307 is attached only to the region of one surface of the membrane filter 200, which does not adjoin the filter support structure 305, i.e., the region exposed toward the movement passages 301.

In case that the salt is attached to the entire lower portion of the membrane, the salt also remains between the membrane filter 200 and the support structure 305. In this case, the salt may be an element that hinders the bonding region of the two-sided bonding member 306 to the extent of the space of the salt. The separated plasma may melt the salt and adversely affect the bonded state in the future.

In addition, in case that the salt is attached only to the region exposed toward the movement passages 301 without being attached to the entire lower portion as disclosed in the present specification, it is possible to assist in guiding the flow of the fluid in a particular direction (toward the main channel) .

In addition, the inside of the sub-channel needs to be filled with the separated fluid (plasma) so that the separated fluid (plasma) may flow to the main channel 302. In case that droplets are produced in a '¬'-shaped gap in a space between the lower portion of the membrane and a wall surface of the support structure because the salt is applied between the lower portion of the membrane and the wall surface of the support structure when the separated fluid wets the salt matrix on one surface of the membrane, there is a problem in that the sub-channels 301 cannot be filled with the fluid before the fluid meets the droplets on the wall surface at the opposite side. In contrast, in case that the salt is attached only to the region exposed toward the movement passages 301 without being attached to the entire lower portion as disclosed in the present specification, the droplets are produced only on the lower portion of the membrane without coming into contact with the wall surface, such that the droplets are greatly grown and come into contact with any one of the three surfaces of the lower portion and then come into contact with the remaining surfaces, which may increase a speed at which the channel is filled with the fluid.

In addition, in case that a positive pressure pump or a negative pressure pump is used to increase a pressure and separate the blood with an enhanced flow, hemolysis may occur in which the red blood cells are damaged. However, the flow pressure of the fluid is increased during the process of separating the blood from the fluid mixture, and the fluid passes through the pressure spike of the membrane without an external pressure of the positive pressure pump or the negative pressure pump, such that quality of the resulting fluid (filtrate, plasma, or serum) may be improved. Likewise, during a process of separating a liquid mixture containing solid particles, the component such as the membrane or the housing may be damaged by the pressure, or a desired separation result cannot be obtained. However, in case that the fluid flows without the external pressure of the positive pressure pump or the negative pressure pump, there is an advantage in that the internal components such as the membrane or the housing need not be configured to cope with the high pressure.

Meanwhile, in case that the membrane protrudes or is fixed onto an etched lower support structure, i.e., the salt is applied onto a part or the entirety of the lower portion of the membrane in a state in which the membrane is fixed to a planar body having no structure, particular directionality does not occur between the lower portion of the membrane and the body, which makes it difficult to introduce the fluid into the main channel. However, in case that the salt is applied onto the lower portion of the membrane, particularly, an edge or a periphery thereof, the separated fluid wets the lateral surface of the membrane. Therefore, a flow of the non-separated fluid may occur along a gap between the membrane and the body. That is, particular directionality may occur so that the separated fluid flows toward the main channel.

In the two-sided bonding member 306, an area of the exposed region of one surface of the membrane filter 200 may be smaller than an area of the upper portion of the filter support structure 305, such that the salt matrix is more assuredly attached only to the region of one surface of the membrane filter 200 that is exposed toward the movement passages. In other words, an area of the two-sided bonding member 306 attached to the membrane filter 200 may be larger than an area of the upper portion of the filter support structure 305.

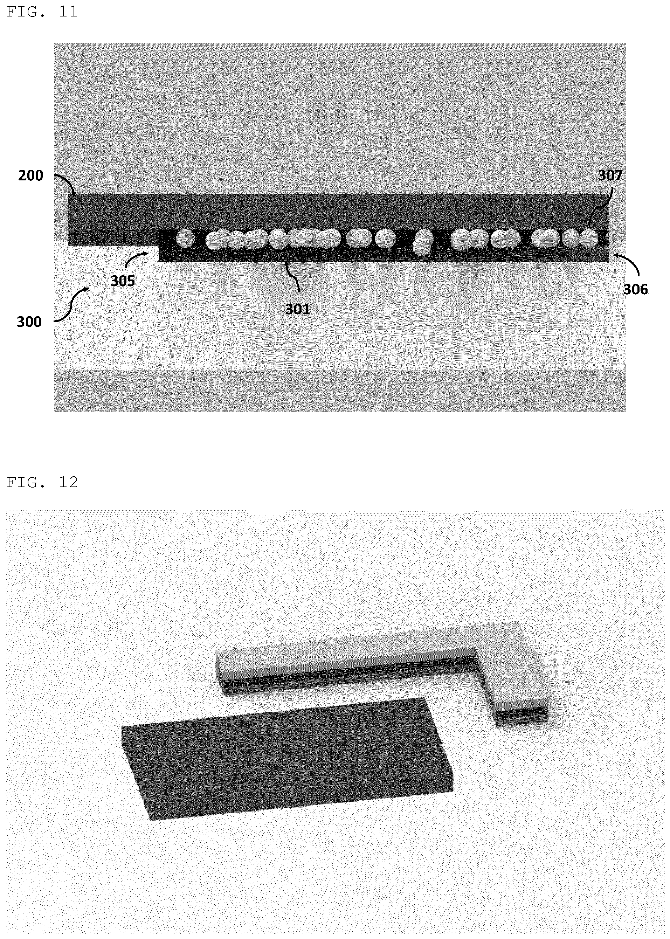

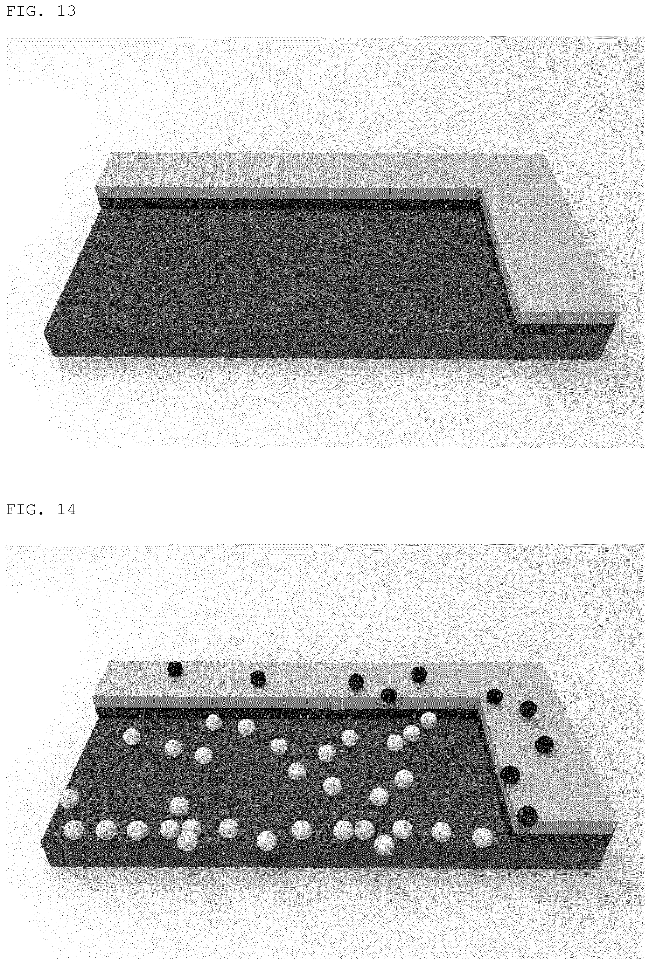

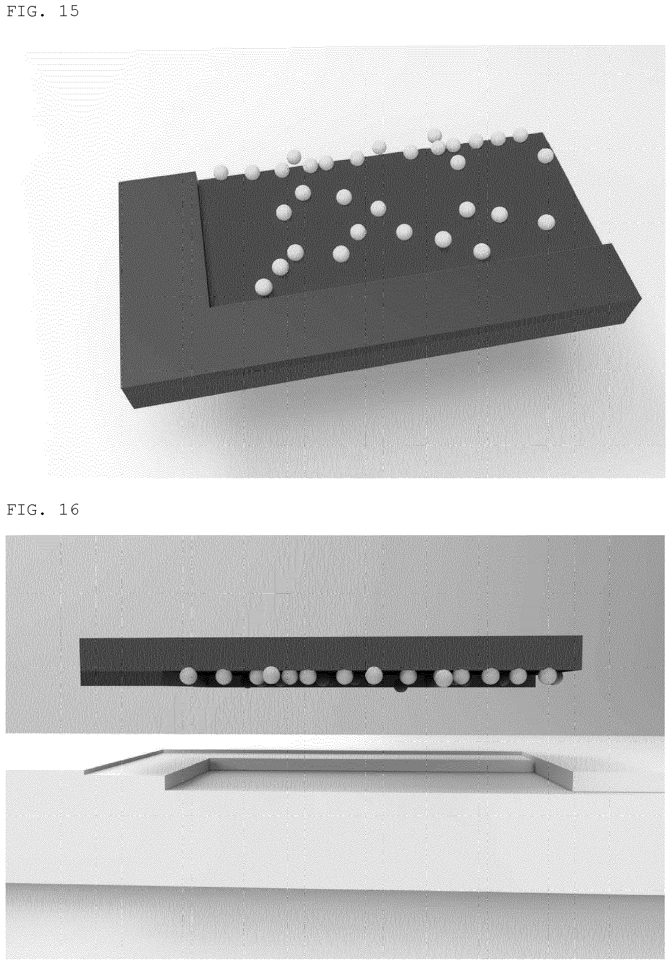

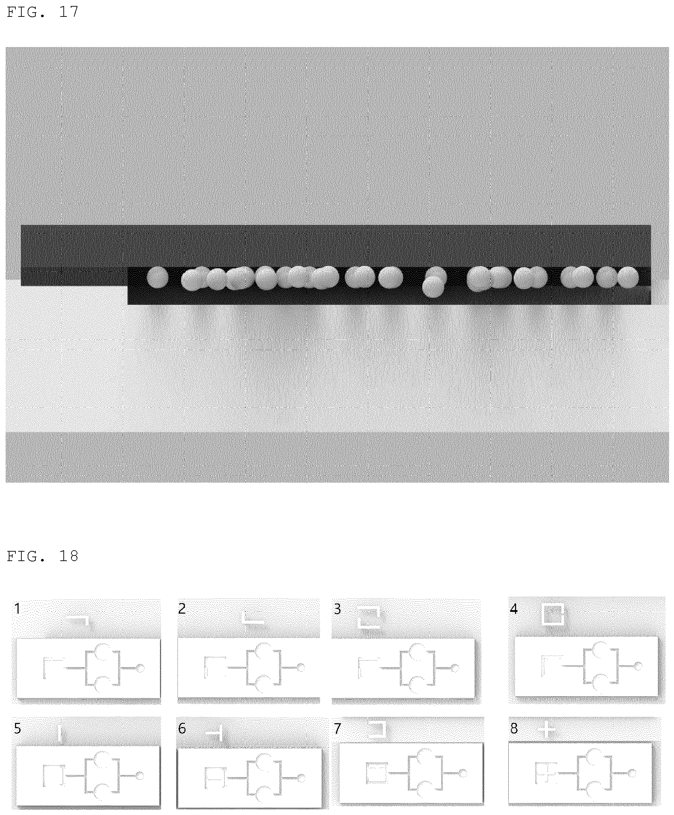

Meanwhile, FIGS. 10 to 17 are views illustrating other embodiments related to the microfluidic device and the method of mounting the membrane filter according to the present specification. Referring to the comparison between FIG. 10 and FIG. 1, it can be ascertained that the filter support structure illustrated in FIG. 10 differs from that in the embodiment illustrated in FIG. 1. That is, the embodiment illustrated in FIG. 10 is an embodiment in which the shape of the filter support structure is simplified to a '¬' shape in comparison with the embodiment illustrated in FIG. 1. Further, FIG. 11 corresponds to FIG. 2, FIG. 12 corresponds to FIG. 3, FIG. 13 corresponds to FIG. 4, FIG. 14 corresponds to FIG. 6, FIG. 15 corresponds to FIG. 7, FIG. 16 corresponds to FIG. 8, and FIG. 17 corresponds to FIG. 9. FIGS. 10 to 17 illustrate that the microfluidic device and the method of mounting the membrane filter according to the present specification are not limited by the shape of the filter support structure and/or the size and shape of the movement passage.

FIG. 18 is a view illustrating various embodiments of a protrusion pattern.

Referring to FIG. 18, it can be ascertained that the protrusion patterns on which the membrane filter may be seated are formed in various shapes. The protrusion pattern may also serve to define the movement passage while serving to support the membrane. Therefore, it should be understood from the above-mentioned examples that the protrusion pattern according to the present specification may have various shapes without being limited to the above-mentioned examples.

While the embodiments of the present specification have been described with reference to the accompanying drawings, those skilled in the art will understand that the present specification may be carried out in any other specific form without changing the technical spirit or an essential feature thereof. Therefore, it should be understood that the above-described embodiments are illustrative in all aspects and do not limit the present disclosure.

## Claims

1. A method of mounting a membrane filter, the method comprising:
(a) providing a two-sided bonding member having a bonding matrix with a bonding force at two opposite surfaces thereof and a protective film attached on one of the two opposite surfaces ;
(b) attaching the two-sided bonding member to one surface of a membrane filter having desired filtering properties, in a state in which a partial region of one surface of the membrane filter to which the two-sided bonding member is attached is exposed;
(c) applying salt onto one surface of the membrane filter to which the two-sided bonding member is attached;
(d) removing the protective film of the two-sided bonding member attached to the membrane filter; and
(e) aligning the membrane filter so that the two-sided bonding member, on which a bonding agent exposed from the removed protection film, corresponds to a filter support structure, and bonding the membrane filter to the filter support structure.

2. The method of claim 1, wherein a shape of a non-exposed region of one surface of the membrane filter to which the two-sided bonding member is attached corresponds to a shape of the filter support structure.

3. The method of claim 1 or 2, wherein in the two-sided bonding member provided in (a), a shape of the bonding matrix corresponds to a shape of the filter support structure.

4. The method of anyone of claims 1 to 3, wherein (b) is attaching the two-sided bonding member to a region of one surface of the membrane filter that corresponds to a shape of the filter support structure.

5. The method of anyone of claims 1 to 4, wherein (b), an area of an exposed region of one surface of the membrane filter is smaller than an area of an upper portion of the filter support structure.

6. The method of anyone of claims 1 to 5, wherein (c) further comprises applying the salt by using a method of diffusing an intermediate medium and drying the salt.

7. A microfluidic device comprising:
a microfluid channel provided in a body;
a sample input port formed in the body and provided in a region adjacent to one end of the microfluid channel;
a reaction chamber formed in the body and connected to the other end of the microfluid channel;
a membrane filter disposed between the sample input port and the microfluid channel and having desired filtering properties;
a filter support structure provided between the membrane filter and the microfluid channel and configured to support the membrane filter, the filter support structure having one or more protrusion patterns that define movement passages so that a fluid separated from a sample injected through the sample input port is collected in the microfluid channel; and
a bonding member disposed between the membrane filter and upper surfaces of the one or more protrusion patterns,
wherein a salt matrix is attached to a region of a surface of the membrane filter that adjoins the filter support structure and is exposed toward the movement passage.

## Amended claims

### Amended claims in accordance with Rule 137(2) EPC.

1. A method of mounting a membrane filter, the method comprising:
(a) providing a two-sided bonding member (306) having a bonding matrix with a bonding force at two opposite surfaces thereof and a protective film attached on one of the two opposite surfaces,
wherein a shape of the bonding matrix corresponds to a shape of a filter support structure (305) having a plural of protrusion patterns that define movement passages (301) so that a fluid separated from a sample injected through the sample input port (101) is collected in the microfluid channel (301, 302, 303, 304);
wherein the filter support structure (305) is provided between the membrane filter (200) and the microfluid channel (301, 302, 303, 304) and serve to support the membrane filter (200);
(b)attaching the two-sided bonding member (306) to one surface of a membrane filter (200) having desired filtering properties,
wherein attaching the two-sided bonding member (306) to only a region of one surface of the membrane filter (200) that corresponds to a shape of the filter support structure (305), in a state in which a partial region of one surface of the membrane filter (200) to which the two-sided bonding member (306) is attached is exposed and a shape of a non-exposed region of one surface of the membrane filter (200) to which the two-sided bonding member (306) is attached corresponds to a shape of the filter support structure (305);
(c)applying salt onto one surface of the membrane filter (200) to which the two-sided bonding member (306) is attached;
(d)removing the protective film of the two-sided bonding member (306) attached to the membrane filter (200); and
(e)aligning the membrane filter (200) so that the two-sided bonding member (306), on which a bonding agent exposed from the removed protection film, corresponds to the filter support structure (305), and bonding the membrane filter (200) to the filter support structure (305).

2. The method of claim 1, wherein (b), an area of an exposed region of one surface of the membrane filter (200) is smaller than an area of an upper portion of the filter support structure (305).

3. The method of claim 1, wherein (c) further comprises applying the salt by using a method of diffusing an intermediate medium and drying the salt.

4. A microfluidic device comprising:
a microfluid channel (301, 302, 303, 304)provided in a body;
a sample input port (101) formed in the body and provided in a region adjacent to one end of the microfluid channel (301, 302, 303, 304);
a reaction chamber (303) formed in the body and connected to the other end of the microfluid channel (301, 302, 303, 304);
a membrane filter (200) disposed between the sample input port (101) and the microfluid channel (301, 302, 303, 304)and having desired filtering properties;
a filter support structure (305) provided between the membrane filter (200) and the microfluid channel (301, 302, 303, 304)and configured to support the membrane filter (200), the filter support structure (305) having a plural of protrusion patterns that define movement passages (301) so that a fluid separated from a sample injected through the sample input port (101) is collected in the microfluid channel (301, 302, 303, 304); and
a bonding member (306) disposed between the membrane filter (200) and upper surfaces of the one or more protrusion patterns,
wherein a salt matrix is attached to only a region of a surface of the membrane filter (200) that adjoins the filter support structure (305) and is exposed toward the movement passage (301) .
